# EUROPEAN PATENT APPLICATION

(11) **EP 3 549 945 A1**
(43) Date of publication of application: **09.10.2019**
(21) Application number: 17878651.3
(22) Date of filing: 05.12.2017
(51) Int. Cl.: C07J 41/00, C07J 43/00, C07J 51/00, C07J 7/00, A61K 31/57, A61K 31/58, A61K 31/662, A61P 25/00, A61P 25/20, A61P 37/06, A61P 35/00, A61P 25/08

(54) **WATER-SOLUBLE ALLOPREGNENOLONE DERIVATIVE AND USE THEREOF**

(30) Priority: 05.12.2016 CN 201611101726
(71) Applicant: Jiangsu Nhwaluokang Pharmaceutical Research and Development Co., Ltd., Xuzhou City, Jiangsu 221000 (CN)
(72) Inventor: LI, Qingeng, Chongqing 400016 (CN); WANG, Tao, Chongqing 400016 (CN); CHEN, Gang, Chongqing 400016 (CN); REN, Liang, Chongqing 400016 (CN); LIU, Xin, Chongqing 400016 (CN)
(74) Representative: Turner, Craig Robert
(86) International application number: PCT/CN2017/114590
(87) International publication number: WO 2018/103626

(57) **Abstract**

The present invention relates to the field of medicine, and particularly relates to a water-soluble allopregnenolone (3β,5α-tetrahydroprogesterone) derivative, a pharmaceutical composition comprising the same, and use thereof in prevention or treatment of central nervous system diseases, in sedation and hypnosis, in treatment of Alzheimer's disease, in treatment of epilepsy or in treatment of depression, especially postpartum depression.

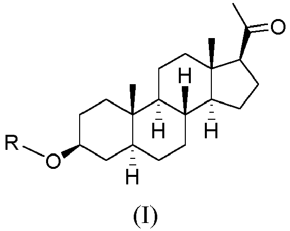

## Description

### CROSS-REFERENCE TO RELATED APPLICATIONS

The present application claims the benefit of priority from Chinese patent application No. 201611101726.X filed on December 5, 2016, the contents of which are incorporated herein by reference in its entirety.

### TECHNICAL FIELD

The present invention relates to the field of medicines, and particularly to a class of water-soluble allopregnenolone (3β,5α-tetrahydroprogesterone) derivatives, a pharmaceutical composition comprising the same, and use thereof in prevention or treatment of central nervous system diseases, in sedation and hypnosis, in treatment of Alzheimer's disease, in treatment of epilepsy or in treatment of depression, especially postpartum depression.

### BACKGROUND ART

Neurosteroids play an important physiological role in human body. Damages to the synthesis of neurosteroids *in vivo* will cause different neurological (CN 104736158 A) or mental (Expert Opin Ther Targets. 2014; 18(6):679-90) diseases. There are a wide range of neurosteroids, among which allopregnanolone is a GABA (γ-aminobutyric acid) receptor agonist in human body that can be bound to GABA so as to exert physiological effects (J Physiol. 2001; 537(Pt 2): 453-465). Scientists have made a great deal of studies on the therapeutic use of allopregnanolone (e.g., for treating nervous system diseases such as Alzheimer's disease (1. Alzheimer's Assoc Int Conf (AAIC), 2013, Abst P4-012; and 2. 12th IntConf Alzheimer Parkinson Dis (AD/PD), 2015, Abst 155), epilepsy (1. Epilepsia 2013, 54: 81; 2. 68th Annu Meet Am Acad Neurol (AAN), 2016, Abst P4.210; and 3. 68th Annu Meet Am Acad Neurol (AAN), 2016, Abst S14.003), postpartum depression (DailyDrugNews.com, June 11, 2015), etc.).

Chinese patent publication No. CN 104736158 A has disclosed a composition comprising allopregnanolone and cyclodextrin, and a method for treating epilepsy or continuous epilepsy by intravenous infusion of the composition. In the allopregnanolone-cyclodextrin composition of this patent publication, cyclodextrin accounts for 1∼30% and has a blood concentration of 50 ∼ 2,300 nM, and the treatment period of the composition is up to more than 24 hours. However, cyclodextrin, as a polymeric compound, has a certain risk of nephrotoxicity (Safety Study on Cyclodextrin Derivatives, a Supermolecular Material Used in Medicines, Chinese Materials Science Technology & Equipment, No. 5, 2009, pp. 1-3), and thus has potential safety hazards.

As a steroid compound, allopregnenolone has lower solubility in water. Therefore, it is quite necessary to develop allopregnenolone derivatives having improved water solubility and safety.

### SUMMARY OF THE INVENTION

In one aspect, the present invention provides a water-soluble allopregnenolone derivative. The water-soluble allopregnenolone derivative has good physical/chemical stability and good water solubility, and furthermore can be dissociated rapidly *in vivo* to release the active drug (allopregnenolone), thus providing pharmacological effects quickly.

The water-soluble allopregnenolone derivative of the present invention is a compound of formula (I), or a pharmaceutically acceptable salt, a stereoisomer, a polymorph, or a solvate thereof: 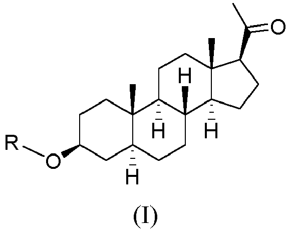 wherein 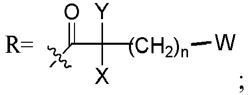
X is H or F;
Y is H, F, or C₁₋₆ alkyl optionally substituted with one or more F;
n is 0, 1, 2, 3, 4, 5 or 6;
W is W¹ or W²;
W¹ is NR¹R²•A or 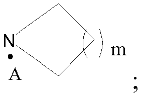
R¹ and R² are each independently H, C₁₋₆ alkyl optionally substituted with phenyl, or C₃₋₆ cycloalkyl;
m is 0, 1, 2 or 3;
A is absent or is a pharmaceutically acceptable acid;
W² is -COOH, -OPO₃(H)₂, -PO₃(H)₂, -COO(M)_{1/t}, -OPO₃(M)_{2/t} or -PO₃(M)_{2/t};
M is a metal ion, an ammonium ion or a basic amino acid cation; and
t is the charge number of M.

In a further aspect, the present invention provides a pharmaceutical composition comprising a prophylactically or therapeutically effective amount of the compound of formula (I) of the present invention, or a pharmaceutically acceptable salt, a stereoisomer, a polymorph, or a solvate thereof, and one or more pharmaceutically acceptable carriers.

In a further aspect, the present invention provides use of the compound of formula (I) of the present invention or a pharmaceutically acceptable salt, a stereoisomer, a polymorph, or a solvate thereof or the pharmaceutical composition of the present invention in the manufacture of a medicament for preventing or treating central nervous system diseases, for sedation and hypnosis, for treating Alzheimer's disease, for treating epilepsy, or for treating depression, especially postpartum depression.

In a further aspect, the present invention provides the compound of formula (I) of the present invention, or a pharmaceutically acceptable salt, a stereoisomer, a polymorph, or a solvate thereof, or the pharmaceutical composition of the present invention, for use in prevention or treatment of central nervous system diseases, in sedation and hypnosis, in treatment of Alzheimer's disease, in treatment of epilepsy, or in treatment of depression, especially postpartum depression.

In a further aspect, the present invention provides a method for preventing or treating central nervous system diseases, for sedation and hypnosis, for treating Alzheimer's disease, for treating epilepsy, or for treating depression, especially postpartum depression, comprising administering a prophylactically or therapeutically effective amount of the compound of formula (I) of the present invention, or a pharmaceutically acceptable salt, a stereoisomer, a polymorph, or a solvate thereof, or the pharmaceutical composition of the present invention to a subject in need thereof.

### DETAILED DESCRIPTIONS OF THE INVENTION

### DEFINITIONS

Unless otherwise defined below, all technical and scientific terms and intents used herein should be identical to those commonly understood by those skilled in the art. Techniques used herein refer to those techniques commonly understood in the art, including apparent variations and replacement of equivalent techniques for those skilled in the art. Although we believe the following terms will be well understood by those skilled in the art, the definitions of which are provided herein to better illustrate the present invention.

As used herein, the terms "comprising", "including", "having", "containing" or "relating to" and other variations in this context are inclusive or open-ended and do not exclude other elements, method, or steps not listed.

As used herein, the term "C₁₋₆ alkyl" refers to a saturated, linear or branched hydrocarbon group having 1-6 (e.g., 1, 2, 3, 4, 5 or 6) carbon atoms, such as methyl, ethyl, propyl, isopropyl, *n*-butyl, isobutyl, *sec*-butyl, *tert*-butyl, *n*-pentyl, isopentyl, *neo*-pentyl, *n*-hexyl, isohexyl, etc., preferably methyl, ethyl, propyl, isopropyl, butyl or isobutyl, and more preferably methyl, ethyl or propyl.

As used herein, the term "C₃₋₆ cycloalkyl" refers to a saturated monocyclic hydrocarbon group having 3-6 (e.g., 3, 4, 5 or 6) carbon atoms, such as cyclopropyl, cyclobutyl, cyclopentyl or cyclohexyl.

As used herein, the term "C₆₋₁₄ aryl" refers to an aromatic group having 6 to 14 carbon atoms, such as phenyl or naphthyl.

The pharmaceutically acceptable salts of the compound of the present invention include the salts formed from the compound and pharmaceutically acceptable acids, and the salts formed from the compound and pharmaceutically acceptable bases.

As used herein, the term "pharmaceutically acceptable acid" refers to an acid that can be used in pharmaceuticals, such as hydrochloric acid, hydrobromic acid, sulfuric acid, nitric acid, phosphoric acid, carbonic acid, formic acid, acetic acid, acetoacetic acid, trifluoroacetic acid, propionic acid, pyruvic acid, butyric acid, caproic acid, heptanoic acid, undecanoic acid, lauric acid, stearic acid, palmitic acid, oxalic acid, methanesulfonic acid, trifluoromethanesulfonic acid, ethanedisulfonic acid, isethionic acid, 1,5-naphthalenedisulfonic acid, 2-naphthalenesulfonic acid, camphorsulfonic acid, sulfamic acid, lactic acid, benzenesulfonic acid, *p*-toluenesulfonic acid, malonic acid, succinic acid, glutaric acid, adipic acid, maleic acid, fumaric acid, lactic acid, tartaric acid, citric acid, malic acid, benzoic acid, salicylic acid, cinnamic acid, naphthoic acid, pamoic acid, nicotinic acid, orotic acid, methylsulfuric acid, dodecylsulfuric acid, glutamic acid, aspartic acid, gluconic acid, glucuronic acid, or any combination thereof.

As used herein, the term "pharmaceutically acceptable base" refers to a base that can be used in pharmaceuticals, such as inorganic bases (e.g., alkali metal hydroxide or alkaline earth metal hydroxide, etc.) or organic bases (e.g., amine (primary amine, secondary amine or tertiary amine), etc.). Examples of appropriate salts include, but not limited to, organic salts derived from amino acid, ammonia, primary amine, secondary amine, tertiary amine and cyclamine (e.g., diethylamine salt, piperidine salt, morpholine salt, piperazine salt, choline salt, meglumine salt, tromethamine salt, etc.) and inorganic salts derived from Na, Ca, K, Mg, Mn, Fe, Cu, Zn, Al and Li.

As used herein, the term "basic amino acid" refers to an amino acid in which OH⁻ generated by hydrolysis is more than H⁺, e.g., arginine, lysine or histidine.

As used herein, the term "stereoisomer" represents isomers formed due to at least one asymmetric center. A compound having one or more (e.g., one, two, three or four) asymmetric centers may exist in the form of a racemic mixture, a single enantiomer, a diastereomeric mixture and a single diastereoisomer.

The compound of the present invention may exist in the form of crystal or polymorph, and may be a single polymorph or a mixture of more than one polymorph in any proportion.

The compound of the present invention may exist in the form of a solvate, especially a hydrate thereof, wherein the compound of the present invention comprises a polar solvent, e.g., water, ethanol, isopropanol, ethyl acetate or acetone, as a structural element of the crystal lattice. The polar solvent, especially water, may exist in a stoichiometric amount or a non-stoichiometric amount.

### COMPOUND

According to an embodiment of the present invention, provided is a water-soluble allopregnenolone derivative, which is a compound of formula (I), or a pharmaceutically acceptable salt, a stereoisomer, a polymorph, or a solvate thereof: 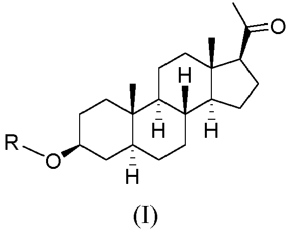 wherein 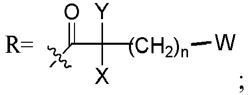
X is H or F;
Y is H, F, or C₁₋₆ alkyl optionally substituted with one or more F;
n is 0, 1, 2, 3, 4, 5 or 6;
W is W¹ or W²;
W¹ is NR¹R²•A or 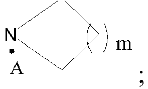
R¹ and R² are each independently H, C₁₋₆ alkyl optionally substituted with phenyl, or C₃₋₆ cycloalkyl;
m is 0, 1, 2 or 3;
A is absent or is a pharmaceutically acceptable acid;
W² is -COOH, -OPO₃(H)₂, -PO₃(H)₂, -COO(M)_{1/t}, -OPO₃(M)_{2/t} or -PO₃(M)_{2/t};
M is a metal ion, an ammonium ion or a basic amino acid cation; and
t is the charge number of M.

According to an embodiment of the present invention, Y is F, CF₃, CH₂F or CHF₂.

According to an embodiment of the present invention, W is W¹.

According to an embodiment of the present invention, R¹ and R² are each independently C₁₋₆ alkyl optionally substituted with phenyl, e.g., methyl, ethyl, propyl, isopropyl, butyl, isobutyl or benzyl.

According to an embodiment of the present invention, R¹ and R² are each independently C₃₋₆ cycloalkyl, e.g., cyclopropyl, cyclobutyl, cyclopentyl or cyclohexyl.

According to an embodiment of the present invention, R¹ and R² are each independently H, methyl, ethyl, propyl, isopropyl, butyl, isobutyl, benzyl, cyclopropyl, cyclobutyl, cyclopentyl or cyclohexyl.

According to an embodiment of the present invention, R¹ and R² are not H at the same time.

According to an embodiment of the present invention, W¹ is selected from the group consisting of:

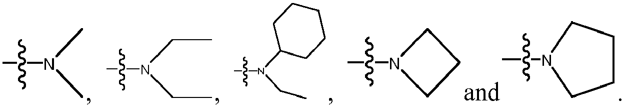

According to an embodiment of the present invention, A is hydrochloric acid, hydrobromic acid, sulfuric acid, nitric acid, phosphoric acid, carbonic acid, formic acid, acetic acid, propionic acid, acetoacetic acid, trifluoroacetic acid, propionic acid, pyruvic acid, butyric acid, caproic acid, heptanoic acid, undecanoic acid, lauric acid, stearic acid, palmitic acid, oxalic acid, methanesulfonic acid, trifluoromethanesulfonic acid, ethanesulfonic acid, ethanedisulfonic acid, isethionic acid, 1,5-naphthalenedisulfonic acid, 2-naphthalenesulfonic acid, camphorsulfonic acid, sulfamic acid, lactic acid, benzenesulfonic acid, *p*-toluenesulfonic acid, malonic acid, succinic acid, glutaric acid, adipic acid, maleic acid, fumaric acid, tartaric acid, citric acid, malic acid, benzoic acid, salicylic acid, cinnamic acid, naphthoic acid, pamoic acid, nicotinic acid, orotic acid, methylsulfuric acid, dodecylsulfuric acid, glutamic acid, aspartic acid, gluconic acid, glucuronic acid, or any combination thereof.

According to an embodiment of the present invention, W is W².

According to an embodiment of the present invention, M is an alkali metal ion (e.g., a lithium ion, a sodium ion or a potassium ion), an alkaline earth metal ion (e.g., a magnesium ion, a zinc ion or a calcium ion) or a trivalent metal ion (e.g., an aluminum ion).

According to an embodiment of the present invention, M is an ammonium ion represented by formula (NR³R⁴R⁵R⁶)⁺ or 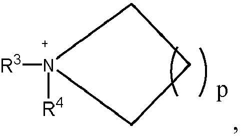 wherein R³ , R⁴, R⁵ and R⁶ are each independently H, alkyl optionally substituted with phenyl, cycloalkyl, or aryl; and p is 0, 1, 2 or 3.

In a preferred embodiment, R³, R⁴, R⁵ and R⁶ are each independently H, C₁₋₆ alkyl optionally substituted with phenyl, C₃₋₆ cycloalkyl, or C₆₋₁₄ aryl. In a more preferred embodiment, R³, R⁴, R⁵ and R⁶ are each independently H, methyl, ethyl, propyl, isopropyl, butyl, isobutyl, phenyl, benzyl, cyclopropyl, cyclobutyl, cyclopentyl or cyclohexyl. In a most preferred embodiment, R³ and R⁴ are each independently H, methyl or ethyl.

In a preferred embodiment, M is selected from

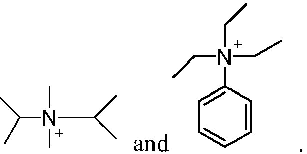

According to an embodiment of the present invention, M is arginine + H⁺, lysine + H⁺ or histidine + H⁺.

In a preferred embodiment, W² is selected from the group consisting of -COONa, -COOK, -P(=O)(OLi)₂, -P(=O)(ONa)₂, -P(=O)(OK)₂, -P(=O)O₂Mg, -OP(=O)(ONa)₂ and -OP(=O)O₂Ca.

In an embodiment, X is different from Y, and the carbon atom to which both X and Y are attached is in a single R configuration, in a single S configuration, or in a mixture of R and S configurations.

The present invention covers compounds obtained by any combination of the embodiments.

According to an embodiment of the present invention, variable R in the compound of the present invention is selected from the group consisting of:

| Compound No. | R | Compound No. | R |
|---|---|---|---|
| Compound 1 | 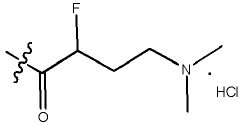 | Compound 2 | 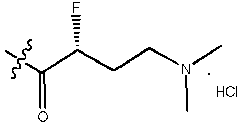 |
| Compound 3 | 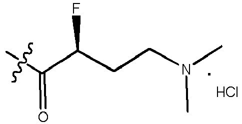 | Compound 4 | 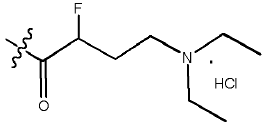 |
| Compound 5 | 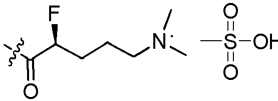 | Compound 6 | 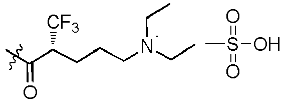 |
| Compound 7 | 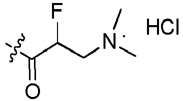 | Compound 8 | 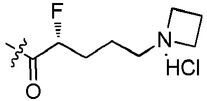 |
| Compound 9 | 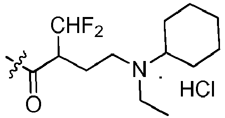 | Compound 10 | 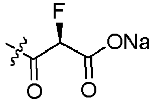 |
| Compound 11 | 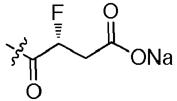 | Compound 12 | 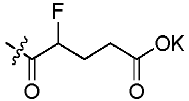 |
| Compound 13 | 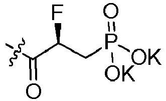 | Compound 14 | 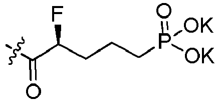 |
| Compound 15 | 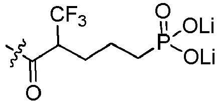 | Compound 16 | 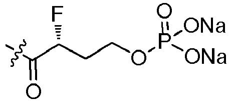 |
| Compound 17 | 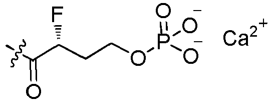 | Compound 18 | 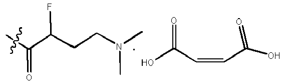 |
| Compound 19 | 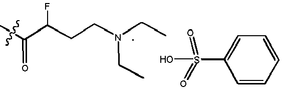 | Compound 20 | 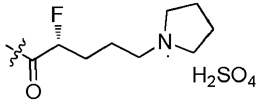 |
| Compound 21 | 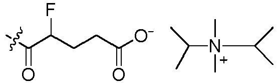 | Compound 22 | 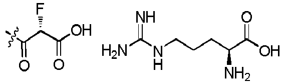 |
| Compound 23 | 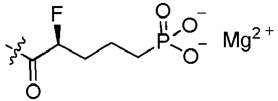 | Compound 24 | 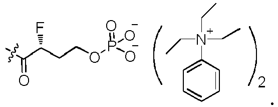 |

### PHARMACEUTICAL COMPOSITION

According to a further embodiment of the present invention, provided is a pharmaceutical composition comprising a prophylactically or therapeutically effective amount of the compound of formula (I) of the present invention, or a pharmaceutically acceptable salt, a stereoisomer, a polymorph, or a solvate thereof, and one or more pharmaceutically acceptable carriers.

The term "pharmaceutically acceptable carrier" in the present invention refers to a diluent, an adjuvant, an excipient or a vehicle which is administrated together with a therapeutic agent, and within the scope of sound medical judgment is suitable for contact with humans and/or other animal tissues without undue toxicity, irritation, allergic reactions or with a reasonable benefit / risk ratio relative to its problems or complications.

The pharmaceutically acceptable carrier in the pharmaceutical composition of the present invention include, but not limited to, sterile liquids, such as water and oils, including those of petroleum, animal, vegetable or synthetic origin, such as peanut oil, soybean oil, mineral oil, sesame oil and the like. When the pharmaceutical composition is administered intravenously, water is an exemplary carrier. Saline, glucose, and glycerol solution can also be used as liquid carriers, particularly for injectable solutions. Suitable pharmaceutical excipients include starch, glucose, lactose, sucrose, gelatin, maltose, chalk, silica gel, sodium stearate, glycerol monostearate, talc, sodium chloride, dried skim milk, glycerol, propylene glycol, water, ethanol, etc. The composition, if desired, may also contain minor amounts of wetting agents, emulsifying agents (e.g., Tween-80, etc.), or pH buffering agents.

The pharmaceutical composition of the present invention may be administrated via suitable routes. Preferably, the pharmaceutical composition of the present invention is administrated orally, intravenously, intraarterially, subcutaneously, intraperitoneally, intramuscularly or transdermally.

The composition of the present invention may be administered in an appropriate dosage form for these administration routes.

The dosage forms include, but not limited to, tablets, capsules, troches, dragees, powders, sprays, creams, ointments, suppositories, gels, pastes, lotions, ointments, aqueous suspensions, injectable solutions, elixirs and syrups.

### TREATMENT METHOD AND USE

The compound of the present invention can be used for treating a variety of diseases that can be treated by administering allopregnenolone.

A further embodiment of the present invention provides use of the compound of formula (I) of the present invention or a pharmaceutically acceptable salt, a stereoisomer, a polymorph, or a solvate thereof or the pharmaceutical composition of the present invention in the manufacture of a medicament for preventing or treating central nervous system diseases, for sedation and hypnosis, for treating Alzheimer's disease, for treating epilepsy, or for treating depression, especially postpartum depression.

A further embodiment of the present invention provides the compound of formula (I) of the present invention or a pharmaceutically acceptable salt, a stereoisomer, a polymorph, or a solvate thereof, or the pharmaceutical composition of the present invention, for use in prevention or treatment of central nervous system diseases, in sedation and hypnosis, in treatment of Alzheimer's disease, in treatment of epilepsy, or in treatment of depression, especially postpartum depression.

A further embodiment of the present invention provides a method for preventing or treating central nervous system diseases, for sedation and hypnosis, for treating Alzheimer's disease, for treating epilepsy, or for treating depression, especially postpartum depression, comprising administering a prophylactically or therapeutically effective amount of the compound of formula (I) of the present invention, or a pharmaceutically acceptable salt, a stereoisomer, a polymorph, or a solvate thereof, or the pharmaceutical composition of the present invention to a subject in need thereof.

In a further embodiment of the present invention, the central nervous system diseases are selected from the group consisting of traumatic brain injury, essential tremor, epilepsy (including refractory status epilepticus and rare genetic epilepticus (e.g., Dravet syndrome and Rett syndrome)), depression (including postpartum depression), and Alzheimer's disease.

The term "therapeutically effective amount" as used herein refers to an amount of the compound which will alleviate one or more symptoms of the diseases to be treated to a certain extent after administration.

Dosage regimens may be adjusted to provide the optimum desired response. For example, a single bolus may be administered, several divided doses may be administered over time, or the dose may be proportionally reduced or increased as indicated by the exigencies of the therapeutic situation. It is to be noted that dosage values may vary with the type and severity of the condition to be alleviated, and may include single or multiple doses. It is to be further understood that for any particular subject, specific dosage regimens should be adjusted over time according to the individual need and the professional judgment of the person administering or supervising the administration of the composition.

The amount of the compound of the present invention administered will be dependent on the subject being treated, the severity of the disorder or condition, the rate of administration, the disposal of the compound and the discretion of the prescribing physician. Generally, an effective dosage is in the range of about 0.0001 to about 50 mg per kg body weight per day, for example about 0.01 to about 10 mg/kg/day, in single or divided doses. For a 70 kg human, this would amount to about 0.007 mg to about 3500 mg/day, for example about 0.7 mg to about 700 mg/day. In some instances, dosage levels below the lower limit of the aforesaid range may be more than adequate, while in other cases, still larger doses may be employed without causing any harmful side effect, provided that such larger doses are first divided into several small doses for administration throughout the day.

The content or dosage of the compound of the present invention in the pharmaceutical composition is about 0.01 mg to about 1000 mg, suitably 0.1-500 mg, preferably 0.5-300 mg, more preferably 1-150 mg, particularly preferably 1-50 mg, e.g., 1.5 mg, 2 mg, 4 mg, 10 mg, 25 mg, etc.

Unless otherwise indicated, the term "treating" or "treatment", as used herein, means reversing, alleviating, inhibiting the progress of, or preventing the disorder or condition to which such term applies, or one or more symptoms of such disorder or condition.

As used herein, the term "subject" includes a human or a non-human animal. An exemplary human subject includes a human subject having a disease (such as one described herein) (referred to as a patient), or a normal subject. The term "non-human animal" as used herein includes all vertebrates, such as non-mammals (e.g. birds, amphibians, reptiles) and mammals, such as non-human primates, livestock and/or domesticated animals (such as sheep, dog, cat, cow, pig and the like).

### BENEFICIAL EFFECTS

The water-soluble allopregnenolone derivative of the present invention, which is obtained by structural modification of the hydroxy of allopregnenolone while retaining the pharmacological activity of allopregnenolone, has good physical/chemical stability. After injection of an aqueous solution comprising the allopregnenolone derivatives, the active drug is released *in vivo,* thus exerting pharmacological effects. Surprisingly, the water-soluble allopregnenolone derivative of the present invention has good water solubility, and thus can be formulated into appropriate liquid preparations. Furthermore, the water-soluble allopregnenolone derivative of the present invention can be easily dissociated *in vivo* to release the active drug. Therefore, the water-soluble allopregnenolone derivative of the present invention can improve the water solubility of allopregnenolone, thus reducing the side effects caused by adjuvants.

### EXAMPLES

The present invention will be further described in detail with reference to the following examples for apparency of the purpose and technical solution of the present invention. It should be understood that these examples are provided merely for further illustration of the present invention, but should not be construed as limitation to the scope of the present invention. Any non-essential modifications and/or adjustments to the technical solutions of the present invention by a person skilled in the art based on the above disclosure of the present invention all fall within the protection scope of the present invention.

### Example 1: Compound 1

### Step 1) Preparation of 4-(dimethylamino)-2-fluorobutyryl chloride hydrochloride (intermediate 1)

4-(Dimethylamino)-2-fluorobutyric acid hydrochloride (10 mmol) was placed in thionyl chloride (10 ml), followed by slowly heating to 40°C, and the mixture was allowed to react for 4 hours. Thionyl chloride was removed by evaporation under reduced pressure, and anhydrous dichloromethane (DCM) (15 ml) was added. After stirring, the solvent was removed by evaporation under reduced pressure. Anhydrous dichloromethane (20 ml) was added to the residues, and the solution thus obtained was used for the next step.

### Step 2) Preparation of compound 1

Allopregnenolone (4.5 mmol) and 4-dimethylaminopyridine (DMAP) (8.2 mmol) were dissolved in anhydrous dichloromethane (20 ml) at -78°C, and then a dichloromethane solution of intermediate 1 prepared in step 1) was slowly added dropwise. The reaction was monitored by HPLC. After the reaction was completed, the DCM layer was washed with an aqueous hydrochloric acid solution (pH = about 1.0), and the organic layer was dried over anhydrous sodium sulfate and filtered. The solvent was removed by rotary evaporation, and the residues were purified by preparative chromatography to obtain compound 1. Mass (ESI) [M+H]⁺=450.31.

### Example 2: Compound 2

At room temperature, allopregnenolone (10 mmol), 1-ethyl-(3-dimethylaminopropyl)carbodiimide hydrochloride (80 mmol), (R)-4-(dimethylamino)-2-fluorobutyric acid hydrochloride (70 mmol) and DMAP (1 mmol) were added to anhydrous dichloromethane, and the mixture was heated to reflux under the protection of nitrogen. The reaction was monitored by HPLC. After the reaction was completed, the reaction solution was cooled to room temperature, and was successively washed with an aqueous hydrochloric acid solution (pH = 3, 70 ml^{∗}3) and a saturated brine (70 ml^{∗}1). The organic layer was dried over anhydrous sodium sulfate and filtered. The solvent was removed by rotary evaporation, and the residues were recrystallized with isopropanol to obtain compound 2. Mass (ESI) [M+H]⁺=450.30.

### Example 3: Compound 3

Compound 3 was prepared according to the procedures similar to those in Example 1, except that 4-(dimethylamino)-2-fluorobutyric acid hydrochloride was replaced with (S)-4-(dimethylamino)-2-fluorobutyric acid hydrochloride (10 mmol) in step 1). Mass (ESI) [M+H]⁺=450.31.

### Example 4: Compound 6

Allopregnenolone (R)-5-(diethylamino)-2-(trifluoromethyl)pentanoate hydrochloride was prepared according to the procedures similar to those in Example 1, except that 4-(dimethylamino)-2-fluorobutyric acid hydrochloride was replaced with (R)-5-(diethylamino)-2-(trifluoromethyl)pentanoic acid hydrochloride (10 mmol) in step 1). The hydrochloride salt was dissolved in dichloromethane, and the resulting organic phase was washed with an aqueous solution containing sodium methanesulfonate (adjusted to pH 3 with methanesulfonic acid). The organic layer was dried and filtered, and the solvent was removed by evaporation to obtain compound 6. Mass (ESI) [M+H]⁺=514.32.

### Example 5: Compound 7

Allopregnenolone (4.5 mmol) and 4-dimethylaminopyridine (8.2 mmol) were dissolved in anhydrous dichloromethane (20 ml) at room temperature, and then dicyclohexyl carbodiimide (10 mmol) and 3-(dimethylamino)-2-fluoropropionic acid hydrochloride (8 mmol) were added. The mixture was allowed to react at room temperature, and the reaction was monitored by HPLC. After the reaction was completed, the DCM layer was washed with an aqueous hydrochloric acid solution (pH = about 1.0), and the organic layer was dried over anhydrous sodium sulfate and filtered. The solvent was removed by rotary evaporation, and the residues were purified by preparative chromatography to obtain compound 7. Mass (ESI) [M+H]⁺=436.30.

### Example 6: Compound 11

Allopregnenolone (4.5 mmol) and 4-dimethylaminopyridine (8.2 mmol) were dissolved in anhydrous dichloromethane (20 ml) at -40°C, and a dichloromethane solution of benzyl (R)-4-chloro-3-fluoro-4-oxobutyrate (prepared according to step 1) in Example 1 with (R)-4-(benzyloxy)-2-fluoro-4-oxobutyric acid as a raw material) was slowly added dropwise. The reaction was monitored by HPLC. After the reaction was completed, the DCM layer was washed with an aqueous hydrochloric acid solution (pH = about 1.0), and the organic layer was dried over anhydrous sodium sulfate and filtered. The solvent was removed by rotary evaporation. The resulting product was dissolved in anhydrous tetrahydrofuran (45 ml), and was hydrogenated in the presence of Pd-C catalyst (5-10%, w/w). After hydrogenation, Pd-C was filtered out, the solvent was removed by evaporation, and the residues were purified by preparative chromatography to obtain a corresponding product. A *tert*-butanol solution of an equivalent amount of sodium *tert*-butoxide was slowly added to an anhydrous tetrahydrofuran solution of the resulting product on an ice bath. Solids precipitated out and were filtered, and then the filter cake was washed with a small amount of tetrahydrofuran and dried to obtain compound 11. Mass (ESI) [M-H]⁻=435.23.

### Example 7: Compound 16

Allopregnenolone (4.5 mmol) and 4-dimethylaminopyridine (8.2 mmol) were dissolved in anhydrous dichloromethane (20 ml) at -40°C, and a dichloromethane solution of (R)-dibenzyl (4-chloro-3-fluoro-4-oxobutyl)phosphate (prepared according to step 1) in Example 1 with (R)-4-((bis(benzyloxy)phosphoryl)oxy)-2-fluorobutyric acid as a raw material) was slowly added dropwise. The reaction was monitored by HPLC. After the reaction was completed, the DCM layer was washed with an aqueous hydrochloric acid solution (pH = about 1.0), and the organic layer was dried over anhydrous sodium sulfate and filtered. The solvent was removed by rotary evaporation. The resulting product was dissolved in anhydrous tetrahydrofuran (45 ml), and was hydrogenated in the presence of Pd-C catalyst (5-10%, w/w). After hydrogenation, Pd-C was filtered out, the solvent was removed by evaporation, and the residues were purified by preparative chromatography to obtain a corresponding acid. A *tert*-butanol solution of sodium *tert*-butoxide in an amount equivalent to that of the acid was slowly added to an anhydrous tetrahydrofuran solution of the acid on an ice bath. Solids precipitated out and were filtered, and then the filter cake was washed with a small amount of tetrahydrofuran and dried to obtain compound 16. Mass (ESI) [M+H]⁻=501.22.

### Example 8: Compound 22

Allopregnenolone (4.5 mmol) and 4-dimethylaminopyridine (8.2 mmol) were dissolved in anhydrous dichloromethane (20 ml) at room temperature, and then dicyclohexyl carbodiimide (10 mmol) and (S)-3-(benzyloxy)-2-fluoro-3-oxopropionic acid (8 mmol) were added. The mixture was allowed to react at room temperature, and the reaction was monitored by HPLC. After the reaction was completed, the DCM layer was washed with an aqueous hydrochloric acid solution (pH = about 1.0), and the organic layer was dried over anhydrous sodium sulfate and filtered. The solvent was removed by rotary evaporation. The resulting product was dissolved in anhydrous tetrahydrofuran (30 ml), and was hydrogenated in the presence of Pd-C catalyst (5-10%, w/w). After hydrogenation, Pd-C was filtered out, the solvent was removed by evaporation, and the residues were purified by preparative chromatography to obtain a corresponding acid. An equivalent amount of arginine was added to an anhydrous tetrahydrofuran solution of the acid on an ice bath. Solids precipitated out and were filtered, and then the filter cake was washed with ice-cold tetrahydrofuran to obtain compound 22. Mass (ESI) [M-H]⁻=421.22.

The following compounds were prepared according to methods similar to those of Example 1 or Example 2:

| Compound | Mass |
|---|---|
| 4 | Mass (ESI) [M+H]⁺=487.31 |
| 5 | Mass (ESI) [M+H]⁺=464.33 |
| 8 | Mass (ESI) [M+H]⁺=476.32 |
| 9 | Mass (ESI) [M+H]⁺=532.39 |
| 10 | Mass (ESI) [M-H]⁻=421.22 |
| 12 | Mass (ESI) [M-H]⁻=449.26 |
| 13 | Mass (ESI) [M-2K+H]⁻=471.19 |
| 14 | Mass (ESI) [M-2K+H]⁻=499.22 |
| 15 | Mass (ESI) [M-2Li+H]⁻=549.21 |
| 17 | Mass (ESI) [M-Ca+H]⁻=501.19 |
| 18 | Mass (ESI) [M+H]⁺=450.31 |
| 19 | Mass (ESI) [M+H]⁺=487.30 |
| 20 | Mass (ESI) [M+H]⁺=490.33 |
| 21 | Mass (ESI) [M-H]⁻=449.27 |
| 23 | Mass (ESI) [M+H]⁻=499.17 |
| 24 | Mass (ESI) [M+H]⁻=501.19 |

### Example 9: Stability test of the compounds in a 5% aqueous glucose solution

Each of the compounds to be tested was weighed (1 mg), placed in a 5% glucose solution (2 ml) and formulated into a solution having a concentration of about 0.5 mg/ml. The solution stood at room temperature, and was sampled at several time points (0.5 h, 1 h, 2 h, 3 h and 5 h). The samples were detected by HPLC, and the results were given below:

| Compound No. | Dissociation percent (%) | | | | | | Solution |
|---|---|---|---|---|---|---|---|
| | 0 h | 0.5 h | 1 h | 2 h | 3 h | 5 h | |
| Compound 1 | 0.01 | 0.03 | 0.05 | 0.07 | 0.10 | 0.17 | Clear |
| Compound 2 | 0.01 | 0.03 | 0.04 | 0.06 | 0.11 | 0.17 | Clear |
| Compound 3 | 0.02 | 0.03 | 0.07 | 0.06 | 0.09 | 0.16 | Clear |
| Compound 10 | 0.02 | 0.14 | 0.21 | 0.36 | 0.51 | 0.86 | Clear |
| Compound 11 | 0.02 | 0.11 | 0.19 | 0.32 | 0.49 | 0.79 | Clear |
| Compound 16 | 0.02 | 0.13 | 0.21 | 0.41 | 0.62 | 0.93 | Clear |

The test results show that the dissociation percentages of the tested compounds of the present invention are less than 1% after being placed in a 5% glucose solution (e.g., for 5 h), indicating that the compounds have good stability in a 5% glucose solution and thus are suitable for injection.

### Example 10: Table for solubility of the compounds in water (25°C)

| Compound No. | Solubility (mg/ml) |
|---|---|
| Compound 1 | >0.5mg/ml |
| Compound 2 | >0.5mg/ml |
| Compound 3 | >0.5mg/ml |
| Compound 10 | >0.4mg/ml |
| Compound 11 | >0.4mg/ml |
| Compound 16 | >0.3mg/ml |

It indicates that the compounds provided in the present invention have relatively good solubility in water, thus changing the solubility of allopregnenolone.

### Example 11: Dissociation test of the compounds in whole blood of rats

Each of the compounds to be tested was weighed (1 mg), placed in water (2 ml), and formulated into a solution having a concentration of about 0.5 mg/ml as a stock solution for further use. Whole blood of rats (0.45 ml) was taken and placed in a 2 ml EP tube, which was placed in a 37°C water bath for preheating for 20 seconds. Then the stock solution of the compound (50 µL) was added quickly. After a homogeneous mixing, the EP tube was placed in a 37°C water bath, and time counting was initiated. Acetonitrile (1 ml) was injected immediately at predetermined time points (1 min, 5 min, 10 min, 15 min). The solution was centrifuged for 5 min (15000 rpm), filtered with a 0.22 µm filtration membrane and subjected to HPLC detection, and the results were given below:

| Compound No. | Dissociation percent (%) | | | |
|---|---|---|---|---|
| | 1 min | 3 min | 5 min | 15 min |
| Compound 1 | 43.82 | 65.34 | 81.38 | 100 |
| Compound 2 | 44.40 | 67.17 | 82.41 | 100 |
| Compound 3 | 42.31 | 64.28 | 79.98 | 100 |
| Compound 10 | 39.78 | 52.28 | 72.31 | 100 |
| Compound 11 | 40.83 | 50.35 | 69.32 | 100 |
| Compound 16 | 35.94 | 47.88 | 66.47 | 94.56 |

The test results show that the tested compounds of the present invention can be dissociated in rat plasma to release the active drug (allopregnenolone), and the dissociation percents are more than 90% after 15 min, indicating that the allopregnenolone derivatives provided in the present invention can rapidly release allopregnenolone in the whole blood of rats, thus exerting therapeutic effects.

In addition to those described herein, various modifications to the present invention will be apparent to those skilled in the art based on the foregoing description. Such modifications are intended to fall within the scope of the appended claims. Each reference cited herein (including all patents, patent applications, journal articles, books and any other disclosures) are incorporated herein by reference in its entirety.

## Claims

1. A compound of formula (I), or a pharmaceutically acceptable salt, a stereoisomer, a polymorph, or a solvate thereof: 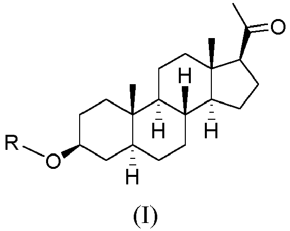 wherein 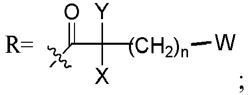
X is H or F;
Y is H, F, or C₁₋₆ alkyl optionally substituted with one or more F;
n is 0, 1, 2, 3, 4, 5 or 6;
W is W¹ or W²;
W¹ is NR¹R²•A or 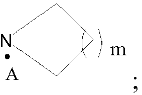
R¹ and R² are each independently H, C₁₋₆ alkyl optionally substituted with phenyl, or C₃₋₆ cycloalkyl;
m is 0, 1, 2 or 3;
A is absent or is a pharmaceutically acceptable acid;
W² is -COOH, -OPO₃(H)₂, -PO₃(H)₂, -COO(M)_{1/t}, -OPO₃(M)₂/t or -PO₃(M)_{2/t};
M is a metal ion, an ammonium ion or a basic amino acid cation; and
t is the charge number of M.

2. The compound of formula (I) according to claim 1, or a pharmaceutically acceptable salt, a stereoisomer, a polymorph, or a solvate thereof, wherein Y is F, CF₃, CH₂F or CHF₂.

3. The compound of formula (I) according to claim 1, or a pharmaceutically acceptable salt, a stereoisomer, a polymorph, or a solvate thereof, wherein when X is different from Y, the carbon atom to which both X and Y are attached is in a single R configuration, in a single S configuration, or in a mixture of R and S configurations.

4. The compound of formula (I) according to claim 1, or a pharmaceutically acceptable salt, a stereoisomer, a polymorph, or a solvate thereof, wherein R¹ and R² are each independently H, methyl, ethyl, propyl, isopropyl, butyl, isobutyl, benzyl, cyclopropyl, cyclobutyl, cyclopentyl or cyclohexyl.

5. The compound of formula (I) according to claim 1, or a pharmaceutically acceptable salt, a stereoisomer, a polymorph, or a solvate thereof, wherein the metal ion is a lithium ion, a sodium ion, a potassium ion, a magnesium ion, a zinc ion, a calcium ion or an aluminum ion.

6. The compound of formula (I) according to claim 1, or a pharmaceutically acceptable salt, a stereoisomer, a polymorph, or a solvate thereof, wherein the ammonium ion is (NR³R⁴R⁵R⁶)⁺ or 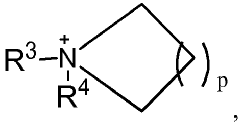 wherein R³, R⁴, R⁵ and R⁶ are each independently H, C₁₋₆ alkyl optionally substituted with phenyl, C₃₋₆ cycloalkyl, or C₆₋₁₄ aryl; and p is 0, 1, 2 or 3.

7. The compound of formula (I) according to claim 6, or a pharmaceutically acceptable salt, a stereoisomer, a polymorph, or a solvate thereof, wherein R³, R⁴, R⁵ and R⁶ are each independently H, methyl, ethyl, propyl, isopropyl, butyl, isobutyl, phenyl, benzyl, cyclopropyl, cyclobutyl, cyclopentyl or cyclohexyl.

8. The compound of formula (I) according to claim 1, or a pharmaceutically acceptable salt, a stereoisomer, a polymorph, or a solvate thereof, wherein the basic amino acid cation is arginine + H⁺, lysine + H⁺ or histidine + H⁺.

9. The compound of formula (I) according to claim 1, or a pharmaceutically acceptable salt, a stereoisomer, a polymorph, or a solvate thereof, wherein R is selected from the group consisting of:
| Compound No. | R | Compound No. | R |
|---|---|---|---|
| Compound 1 | 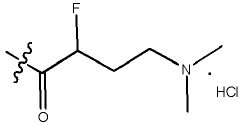 | Compound 2 | 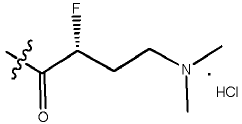 |
| Compound 3 | 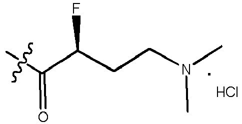 | Compound 4 | 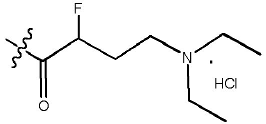 |
| Compound 5 | 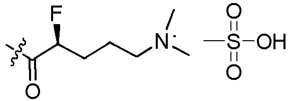 | Compound 6 | 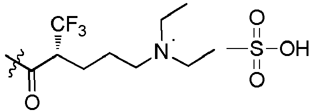 |
| Compound 7 | 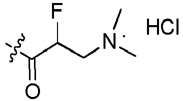 | Compound 8 | 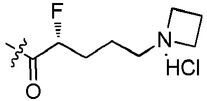 |
| Compound 9 | 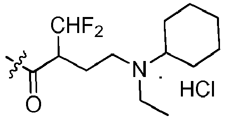 | Compound 10 | 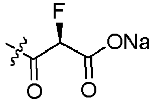 |
| Compound 11 | 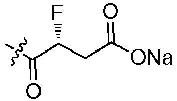 | Compound 12 | 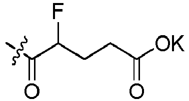 |
| Compound 13 | 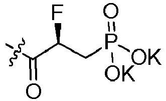 | Compound 14 | 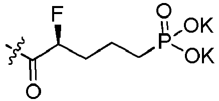 |
| Compound 15 | 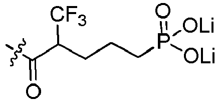 | Compound 16 | 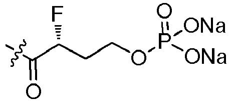 |
| Compound 17 | 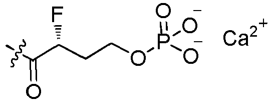 | Compound 18 | 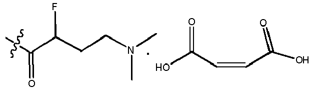 |
| Compound 19 | 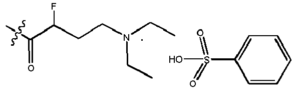 | Compound 20 | 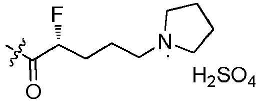 |
| Compound 21 | 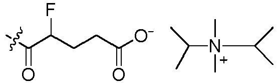 | Compound 22 | 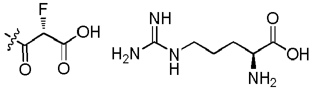 |
| Compound 23 | 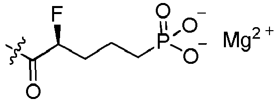 | Compound 24 | 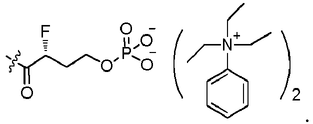 |

10. A pharmaceutical composition comprising a prophylactically or therapeutically effective amount of the compound of formula (I) according to any one of claims 1-9, or a pharmaceutically acceptable salt, a stereoisomer, a polymorph, or a solvate thereof, and one or more pharmaceutically acceptable carriers.

11. Use of the compound of formula (I) according to any one of claims 1-9 or a pharmaceutically acceptable salt, a stereoisomer, a polymorph, or a solvate thereof or the pharmaceutical composition according to claim 10 in the manufacture of a medicament for preventing or treating central nervous system diseases, for treating Alzheimer's disease, for treating epilepsy, or for treating depression, especially postpartum depression.

12. The use according to claim 11, wherein the central nervous system diseases are selected from the group consisting of traumatic brain injury, essential tremor, epilepsy (including refractory status epilepticus and rare genetic epilepticus (e.g., Dravet syndrome and Rett syndrome)), depression (including postpartum depression), and Alzheimer's disease.
